# EUROPEAN PATENT APPLICATION

(11) **EP 4 632 126 A1**
(43) Date of publication of application: **15.10.2025**
(21) Application number: 25167557.5
(22) Date of filing: 31.03.2025
(51) Int. Cl.: D04H 1/425, D04H 1/4258, D04H 1/413, D04H 1/46, D04H 1/4282, D04H 1/43, D04H 1/4309, D04H 1/4326, D04H 1/4334, D04H 1/435, D04H 1/74, A61F 13/53

(54) **A NEEDLE-PUNCHED NON-WOVEN FABRIC STRUCTURE FOR LIQUID ABSORPTION APPLICATION AND METHOD OF MANUFACTURING THEREOF**

(30) Priority: 30.03.2024 IN 202421026711
(71) Applicant: Welspun India Limited, Maharashtra Mumbai 400 013 (IN)
(72) Inventor: GOENKA, Dipalai, 400013 MUMBAI (IN); BASU, Basudev, 400013 MUMBAI (IN); ISLAM, Anikul, 400013 MUMBAI (IN); SHAIKH, Aiyub, 400013 MUMBAI (IN); DALVI, Vaibhav, 400013 MUMBAI (IN); LAHA, Animesh, 400013 MUMBAI (IN); SHARMA, Rajeev, 400013 MUMBAI (IN); SHUKLA, Prateek, 400013 MUMBAI (IN)
(74) Representative: Finnegan Europe LLP

(57) **Abstract**

The present invention is intended for use in producing non-woven fabric structure from either natural fibers or synthetic fibers or a blend of natural and synthetic fibers, the produced non-woven fabrics being especially desirable for use in liquid absorbent application. Also, the present invention may be used in producing a non-woven fabric from two or more webs of loosely matted fibers having a scrim of woven, non-woven, or bonded fabric being interposed between the webs or batts of loosely matted fibers. The primary method of forming the said fabric from the different materials is through needle punched pressing to render high water absorption and retention along with strength. The present invention provides an innovative needle punched nonwoven structured fabric of GSM ranges from 50 to 1000 or more in combination of different fibers. The fiber could be natural fibers or from synthetic origin in appropriate ratio as per the requisite end application.

## Description

### FIELD OF INVENTION

The present invention relates to a needle punched non-woven fabric structure for liquid absorption and method of manufacturing thereof. More particularity, present invention provides the needle punched fabric structure having liquid retention capacity along with high strength.

### BACKGROUND OF INVENTION

Over the past few years, there has been notable progress in the advancement of polymeric materials. These materials offer versatility for a wide range of applications. Among the notable applications, the textile industry stands out. Specifically, the technique of melt spinning of thermoplastic synthetic materials to create continuous filaments, staple fibers, and yarns has brought about significant changes in textile production.

While the utilization of synthetic filaments has predominantly expanded within knitted or woven fabrics, nonwoven materials composed of synthetic fibers have also witnessed significant growth. Various methods are currently employed for producing nonwoven fabrics from synthetic fibers, as well as blends of natural and synthetic fibers. These nonwoven fabrics serve diverse purposes, with a notable application being in carpet manufacturing. Synthetic nonwoven fabrics are particularly favoured for carpet backing due to their resistance to mildew-induced deterioration. Consequently, carpets incorporating synthetic nonwoven backings are highly suitable for use in moisture-prone environments such as patios and outdoor areas.It is observed needle-punched materials have found a wide range of applications in various industries that includes filters, insulation to upholstery, thermal insulation, acoustic control, geotextiles and automotive,

The majority of fabrics are woven, a process conducted on a loom where interlocking warp (the thread or fiber running lengthwise on the loom) and weft (the thread crossing the warp and interlocking with it) fibers form a flat fabric piece.

Nonwoven fabric differs from traditional woven or knitted cloth in its manufacturing process. Instead of being formed through the interlacing of yarns, nonwoven fabric is created by entangling short or long staple fibers of synthetic or manmade fiber or natural fibers or any suitable combination thereof in a random or directional manner to form a web structure, which is then bonded together mechanically, thermally, or chemically. This innovative approach represents a departure from conventional textile methods, offering advantages such as a streamlined production process, rapid output, cost-effectiveness, versatility in raw material selection, and accessibility. Nonwoven fabric also boasts favourable characteristics including good filterability, permeability, and absorption, making it ideal for use as a filter medium in applications such as bag filters and cartridge filters.

Two distinct challenges arise when striving to create thin, flexible, garment-like products with exceptional absorbency. The first challenge involves ensuring that the core system possesses adequate fluid storage capacity to accommodate larger discharge volumes. The second challenge entails preserving the shape and fluid storage capacity of the product when subjected to bodily compressive forces during wear.

In the past, highly absorbent products like those for managing incontinence or heavy menstrual flow tended to be thicker to rapidly absorb large amounts of discharge. Which leads to user discomfort. However, newer developments have led to thinner products with high absorbency. Nonetheless, these thinner products typically possess greater stiffness and resistance to deformation in order to retain their original shape and uphold core storage volume when subjected to pressure.

These types of products face an additional constraint: when subjected to bodily compressive forces, they have a tendency to buckle or undergo plastic deformation. As a result, the garment may lose its ability to exert adequate recovery force, typically facilitated by elastics and material stretch during movement. This lack of recovery force prevents the product from returning to its intended shape, which is essential for optimal fluid absorption and maintaining storage volume for fluid retention.

To address these limitations, a solution has been proposed: the utilization of faster absorbent materials that swell upon fluid absorption, such as the rapid superabsorbent polymers commonly found in baby diapers and traditional adult incontinence products.

While these materials offer increased density and expand upon absorption, they tend to be slower in absorbing fluid during instances of high discharge, such as menses or urine. As a result, they often necessitate bulky acquisition volumes to serve as temporary fluid reservoirs, allowing fluid to enter and be held until absorbed by the swellable storage material. However, these acquisition volumes unavoidably contribute to an increase in the thickness of the absorbent articles.

A further challenge encountered with thin and flexible highly absorbent articles is their struggle to maintain the intended shape effectively, thus limiting their ability to optimize fluid absorption rates and sustain a comfortable, body-conforming fit during the user's daily activities. This issue becomes particularly pronounced as the absorbent article becomes saturated after repeated exposure to urine or menses.

In scenarios like stress or early stages of urge incontinence, individuals often wear an absorbent article across multiple instances of discharge. Consequently, it's crucial to maintain the desired "garment-resembling" wearing experience, along with shape stability and absorption properties, even after the product has been loaded. This ensures that women can carry on with their activities without worrying about sagging or noticeable bulges, which are common in thicker products and baby diapers and can potentially lead to embarrassment for the user.

Nonwoven fabrics find application across a broad spectrum of uses, leveraging their tailored properties to advantage. The utilization of specific thermoplastic polymers in fabric construction, along with targeted treatments applied to the fibrous component-whether in its fibrous form or as part of an integrated structure-and the strategic implementation of diverse integration mechanisms, represent typical variables used to fine-tune and enhance the performance of the resulting nonwoven fabric.

Needle-punched nonwoven fabrics are a type of dry method nonwoven fabric. The process relies on the principle of the needle-punch method, which involves using triangular-sectioned (or other cross-sectional) prickers with barbs along their edges to puncture a fiber web. As the barbs penetrate the fiber web, they exert strong short thrusts on the fibers, compressing the originally fluffy fiber web due to the rubbing action between the fibers. When the pricker withdraws from the fiber web, the fiber bundle barbs remain embedded within it, causing many fiber bundles to intertwine and preventing the fiber web from returning to its original fluffy state. Through multiple rounds of needling, numerous fiber bundles are pushed into the fiber web, causing the fibers to become entangled with each other and resulting in the formation of needle-punched nonwoven material with a certain degree of strength and thickness.

Traditional methods and machines used for felting have faced challenges in effectively interlacing and interlooping fibers. This is often due to punching needles not being arranged in a coordinated manner to achieve interlacing and interlooping of fibers from both surfaces of the web or webs being treated. Moreover, conventional machines have typically required multiple passes of the material through the machine to achieve adequate fiber entanglement. Additionally, some machines have been bulky, slow-moving, and cumbersome, which has hindered the entanglement of loosely matted fibers, resulting in products with insufficient strength and density. Furthermore, products made through needle punching have been prone to elongation due to inadequate interlacing and interlooping.

The absence of coherence and uniform napping properties on both surfaces of the resulting product has been a challenge. This deficiency often leads to a loss of strength during subsequent napping operations, particularly when the product is used to make items like blankets.

The technological process involved in manufacturing existing needle-punched nonwoven fabrics is complex, requiring sophisticated equipment. Consequently, the resulting needle-punched nonwoven fabrics often exhibit drawbacks such as excessive thickness, lack of strength, and poor water and air permeability.

Hence, it is imperative to enhance the composition of raw materials utilized in existing needle-punched nonwoven fabrics and refine the manufacturing techniques. By streamlining the manufacturing process, simplifying equipment requirements, and optimizing raw material composition, we can produce needle-punched nonwoven fabrics with improved properties, including increased strength, optimized bulk, and enhanced liquid holding capacity and air permeability.

In an alternative approach to needle-punched fabric formation, market players have observed a simplified method akin to the conventional air forming process. Here, fibers are conveyed to a forming head via an airstream generated by transport fans. The raw materials, whether virgin or recycled fibers, are processed into fiber form using a hammer mill or similar grinding device. Through a suction box positioned beneath a moving foraminous surface, the fibers carried in the airstream are drawn downward onto the surface, creating a fibrous web. At some point in the process, a suitable binder is introduced to the fibers, which is then cured or treated to enhance the integrity of the fibrous web. Subsequently, the resulting web can be further treated or processed in various ways to achieve the desired end product.

The growth of air forming has been fuelled by the strengths and limitations observed across various industries, shaping both product and process advancements. Notably, industries such as papermaking, textiles, and nonwovens have significantly influenced the evolution of air forming technology. With its status as a mature technology and reliance on capital-intensive equipment, the papermaking industry has traditionally prioritized line speed. As some of the initial air forming systems were introduced by papermaking companies, the technology benefited from their emphasis on achieving faster line speeds. Furthermore, papermakers have enjoyed the advantage of utilizing a low-cost raw material, namely wood pulp. Leveraging this cost-effective raw material in early air-formed products has played a crucial role in penetrating new and diverse markets.

Textile manufacturers have played a pivotal role in establishing product standards, particularly in areas such as texture, drape, and permeability, which serve as benchmarks for evaluating nonwoven products. Moreover, the collaboration between the textile and chemical industries has led to the development of a diverse range of synthetic fibers. These synthetic fibers offer enhanced characteristics such as increased strength, resistance to decay, dyeability, and ease of bonding, thereby contributing to the advancement of nonwoven materials.

In general, synthetic textile-type fibers are typically processed using traditional methods like carding and garnetting, which are well-established in the industry. However, textile production lines generally operate at slower speeds compared to papermaking or nonwoven manufacturing processes. Despite this limitation, the versatility and adaptability of air forming systems have allowed nonwoven materials to compete with conventional textile production machinery. This competition is particularly evident in the manufacturing of products such as disposable operating gowns, surgeon's hand towels, and cubicle curtains.

In next phase of development in the absorbent market SAP Superabsorbent polymers were introduced in a variety of chemical forms including natural-based polymer and synthetic polymers. Natural-based polymers include for example agar, caboxyalkyl cellulose, gum, pectin, carboxyalkyl starch, cellulose sulfate, and hydrolysis product of starch acrylonitrile graft polymers. Synthetic polymers include for example polyacrylates, sulfonated polystyrene, polyvinyl alcohol, polyetheylene oxides, polyvinylpyrolidine, polycrylonitriles, polyacrylamide, and hydrolyzed polyacrylamide. While such natural-based absorbent materials are known for use in personnel care products, they have not gained wide usage in such products, because their absorbent properties are generally lower than those of synthetic absorbent materials, such as for example sodium polyacrylate. The relatively high cost of these materials has also hindered their use in consumer absorbent products. Furthermore, natural based superabsorbent materials tend to form soft, gelatinous masses when swollen with liquid. The presence of such gelatinous masses in absorbent products tends to limit liquid transport and distribution within the absorbent article. This phenomenon is known as gel blocking. Gel blocking refers to the situation wherein the particles of superabsorbent material deform during swelling and block the interstitial spaces between particles thus preventing the flow of liquid. Once gel blocking occurs, the product cannot efficiently absorb subsequent insult of liquid, and the absorbent article tends to leak.

For products such as sanitary napkins or diapers, a single layer or multiple layers of absorbent core are typically utilized. Conventionally, this absorbent layer is composed of superabsorbent polymer (SAP) and regenerated cellulose fiber or short-cut fibers, manufactured using Airlay technology. However, a significant drawback of Airlaid layers is their inherent weakness, which can result in decreased production speed during the final product conversion process.

It has been widely recognized to employ nonwoven textile fabrics for a range of applications including disposable diapers, fabric softener sheets, disposable medical garments, automotive trim fabric, and similar products. These nonwoven fabrics are typically manufactured from polymer fibers using various established processes. Typically, these processes involve two main steps: a web forming step to arrange the fibers into a web structure, followed by a web bonding step to interconnect the fibers within the web, creating an integrated structure.

Synthetic superabsorbent polymers come with several drawbacks, including non-biodegradability. Additionally, there is a critical threshold for the amount of superabsorbent material that can be contained within the fiber matrix of the absorbent article. Exceeding this limit can lead to the physical dislodgement of the absorbent material from the cellulosic fibers during manufacturing and transportation. This separation reduces the absorbency of the product and compromises the effectiveness of the superabsorbent material. Moreover, surpassing the specified limit can result in the core failing to function properly, as there may be inadequate liquid wicking and distribution through the storage layer of the absorbent article. Furthermore, such an absorbent core may lack the necessary strength to maintain its dry structure, shape, and integrity.

Synthetic superabsorbent fibers were developed to address these challenges. However, they have encountered several significant obstacles. For instance, superabsorbent fibers are harder to process compared to cellulosic fibers, and they exhibit poor absorbency under load and low tensile strength. Additionally, the cost of these fibers is considerably higher than that of superabsorbent particles. Consequently, despite their potential, superabsorbent polymer fibers have not been widely adopted in absorbent products.

There is a demand for a cellulosic-based superabsorbent fiber that provides the benefits of conventional superabsorbent fibers derived from petrochemicals. Specifically, there is a need for cellulosic-based superabsorbent fibers that do not form soft gelatinous masses upon hydration and exhibit excellent absorbent properties. Additionally, there is a requirement for a straightforward, cost-effective method for producing such fibers. Furthermore, there is a need to introduce an absorbent article featuring an absorbent core utilizing superabsorbent fiber derived from renewable agricultural materials.

It would be advantageous to offer regenerative cellulosic fibrous materials that are chemically bonded with a superabsorbent polymer, resulting in exceptional liquid absorption and retention properties, particularly concerning liquids containing salt. Additionally, there is a need to introduce superabsorbent material in fiber form that combines the superior liquid absorbency capacity of conventional superabsorbent polymers with the excellent liquid distribution properties of cellulosic fibers.

### OBJECT OF THE INVENTION

The main object of the present invention relates to a needle punched non-woven fabric structure for liquid absorption and method of manufacturing thereof.

Another object of the present invention is to provide an innovative needle punched non-woven fabric structure with GSM in the ranges from 50 to 1000 or more in combination of different fibers.

Another objective of the present invention is to provide needle punched non-woven fabric structure having liquid retention capacity along with high strength.

A further object of the invention is to develop absorbent layer for Sanitary Napkin, Diapers, Puppy pads, Patient under pads and other relevant products for single use application.

Another object of the present invention is to provide the needle punched non-woven fabric structure that has an enhanced liquid holding capacity more than 1500% and having high strength in both machine and cross machine direction.

Yet another object of the present invention is to provide optimized entanglement, through needle punched, into the two different type of fiber material (i.e. absorbent fiber and cellulosic fiber) that are generally difficult to keep in one system, due to shape and intrinsic properties, by other means of state of the art.

Still another object of the present invention is to provide a needle punched non-woven fabric structure having blended composition of absorbent fiber : cellulosic fiber in the range of 1:99 to 55:45.

Another object of the present invention is to provide a needle punched non-woven fabric having single layer needle punched nonwoven structured fabric.

Still another object of the invention is to provide a single layer non-woven structure, which can improve the production speed of final product conversion.

Another object of the invention is to produce a fused non-woven fabric having cellulosic fiber with improved dimensional stability and strength as compared to fused non-woven fabrics known in the art.

A further object of the invention is to provide a method of manufacturing non-woven fabric structure that provides more robust and reusable article in terms of strength and absorbance of liquid with reference to conventional methodologies i.e. Air-lay technology.

This summary is provided to introduce a selection of concepts in a simplified form that are further described below in the detailed description. This summary is not intended to identify key features or essential features of the claimed subject matter.

### SUMMARY OF INVENTION

The present invention is intended for use in producing non-woven fabric structure from either natural fibers or synthetic fibers or a blend of natural and synthetic fibers, the produced non-woven fabrics being especially desirable for use in liquid absorbent application. Also, the present invention may be used in producing a non-woven fabric from two or more webs of loosely matted fibers having a scrim of woven, non-woven, or bonded fabric being interposed between the webs or batts of loosely matted fibers. The primary method of forming the said fabric from the different materials is through needle punched pressing to render high water absorption and retention along with strength. The present invention provides an innovative needle punched non-woven structured fabric of GSM ranges from 50 to 1000 or more in combination of different fibers. The fiber could be natural fibers or from synthetic origin in appropriate ratio as per the requisite end application.

### BRIEF DESCRIPTION OF INVENTION

Fig. 1 shows assembly line for needle punched composite fabric production with super absorption polymer and cellulosic fabric in one of the preferred embodiment;

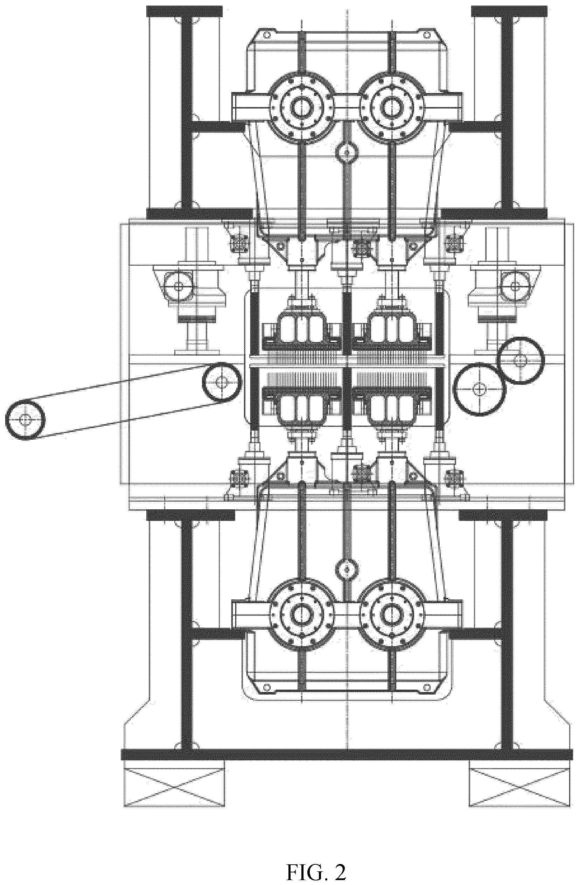

Fig. 2 shows additional assembly line in side perspective view wherein;
Fig. 3 shows calendaring machine in the preferred embodiment; and
Fig. 4 shows graph depicting significance related to one of the object of the invention.
Fig. 5 shows Table-1 showing experimental data for testing data collected for various ratio of absorption fiber and cellulosic fiber composition.

### DETAIL DESCRIPTION OF INVENTION

Before explaining the present invention in detail, it is to be understood that the invention is not limited in its application to the details of the arrangement of parts illustrated in the accompanied drawings. The invention is capable of other embodiments, as depicted in different figures as described above and of being practiced or carried out in a variety of ways. It is to be understood that the phraseology and terminology employed herein is for the purpose of description and not of limitation.

Furthermore, any reference in the specification to "an embodiment" "one embodiment," "various embodiments, or any variant thereof means that a particular feature or aspect of the invention described in conjunction with the particular embodiment is included in at least one embodiment of the present invention. Thus, the appearance of the phrases "in one embodiment," "in another embodiment," or variations thereof in various places throughout the specification are not necessarily all referring to its respective embodiment.

"Absorbent article' refers to devices that absorb and contain body exudates, and, more specifically, refers to devices that are placed against or in proximity to the body of the wearer to absorb and contain the various exudates discharged from the body. Absorbent articles may include diapers, training pants, adult incontinence undergarments, feminine hygiene products, breast pads, care mats, bibs, wound dressing products, and the like. As used herein, the term "body fluids" or "body exudates' includes, but is not limited to, urine, blood, vaginal discharges, breast milk, Sweat and fecal matter.

"Absorbent core" means a structure typically disposed between a top sheet and back sheet of an absorbent article for absorbing and containing liquid received by the absorbent article and may comprise one or more Substrates, absorbent polymer material disposed on the one or more Substrates, and a thermoplastic composition on the absorbent particulate polymer material and at least a portion of the one or more substrates for immobilizing the absorbent particulate polymer material on the one or more Substrates. In a multilayer absorbent core, the absorbent core may also include a cover layer. The one or more Substrates and the cover layer may comprise a nonwoven. Further, the absorbent core is substantially cellulose free. The absorbent core does not include an acquisition system, a topsheet, or a backsheet of the absorbent article. In a certain embodiment, the absorbent core would consist essentially of the one or more substrates, the absorbent polymer material, the thermoplastic composition, and optionally the cover layer.

"Absorbent polymer material", "absorbent gelling material", "AGM", "superabsorbent", and "superabsorbent material" are used herein interchangeably and refer to cross linked polymeric materials that can absorb at least 5 times their weight of an aqueous 0.9% saline solution as measured using the Centrifuge Retention Capacity test.

"Absorbent particulate polymer material is used herein to refer to an absorbent polymer material which is in particulate form so as to be flowable in the dry state. The term can be used inter changeably with "Absorbent polymer material".

"Comprise", "comprising", and "comprises" are open ended terms, each specifies the presence of what follows, e.g., a component, but does not preclude the presence of other features, e.g., elements, steps, components known in the art, or disclosed herein.

"Consisting" essentially of is used hereinto limit the scope of Subject matter, such as that in a claim, to the specified materials or steps and those that do not materially affect the basic and novel characteristics of the Subject matter.

"Disposable" is used in its ordinary sense to mean an article that is disposed or discarded after a limited number of usage events over varying lengths of time, for example, less than about 20 events, less than about 10 events, less than about 5events, or less than about 2 events.

"Diaper" refers to an absorbent article generally worn by infants and incontinent persons about the lower torso so as to encircle the waist and legs of the wearer and that is specifically adapted to receive and contain urinary and fecal waste.

As used herein, term "diaper" also includes "pants".

"Fiber" and "filament" are used interchangeably.

A "nonwoven" is a manufactured sheet, web or batt of directionally or randomly orientated fibers, bonded by friction, and/or cohesion and/or adhesion, excluding paper and products which are woven, knitted, tufted, stitch-bonded incorporating binding yarns or filaments, or felted by wet milling, whether or not additionally needled. The fibers maybe of natural or man-made origin and may be staple or continuous filaments or be formed in situ. Commercially available fibers have diameters ranging from less than about 0.001mm to more than about 0.2 mm and they come in several different forms: short fibers (known as staple, or chopped), continuous single fibers (filaments or monofilaments), untwisted bundles of continuous filaments (tow), and twisted bundles of continuous filaments (yarn). Nonwoven fabrics can be formed by many processes such as melt blowing, spun bonding, solvent spinning, electro-spinning, and carding. The basis weight of nonwoven fabrics is usually expressed in grams per square meter (GSM).

Natural fiber could be viscose, cotton, kapok, nettle, wool, silk, ramie, hemp and synthetic fiber could be absorbent polymer fiber, regenerated cellulose fiber, polyester fiber, nylon fiber with circular or any other suitable cross-section with and without hollowness. These fibers may or may not have functional properties like anti-microbial, anti-fungal, anti-odour, aroma finished.

In some instances, the synthetic fibers could be bio-degradable. This novel non-woven structure can be used as absorbent layer for Sanitary napkin, Diapers, Puppy pads, Patient under pads and other relevant products for single or multiple uses.

In an aspect, the presently claimed invention is directed to a non-woven fabric structure for liquid absorption comprising, at least one fabric core structure containing at least two different fiber components,
(i) first fiber component as cellulosic resource to increase comfort feel during the application; and,
(ii) second fiber component as absorbent polymer resource fiber component to increase water absorption during the application,
wherein, said cellulosic fiber component and absorbent polymer resource fiber component combined together such that a large portion of fabric core have uniformly distributed conjugated bi-component, and wherein the absorbent fiber preferably may have particulate polymer material discontinuously distributed on total fabric core, and the first cellulosic resource fiber component and second absorbent layers are combined together such the each component entangled with another component in uniform way to render fabric core.

In an embodiment, the cellulosic fiber is selected from a group consisting of viscose, cotton, linen, hemp, jute, kapok, ramie, sisal, bamboo, coconut, and mixtures of two or more thereof, more preferably viscose, cotton, linen, hemp, jute, kapok, ramie, sisal, bamboo, and most preferably viscose.

In an embodiment, the absorbent polymer fiber component is selected from a group consisting of polyacrylamide copolymer, ethylene maleic anhydride copolymer, cross-linked carboxymethylcellulose, polyvinyl alcohol copolymers, cross-linked acrylate copolymer, cross-linked polyethylene oxide, starch grafted copolymer of polyacrylonitrile, and mixtures of two or more thereof, preferably cross-linked acrylate copolymer, cross-linked polyethylene oxide, polyacrylamide copolymer, cross-linked carboxymethylcellulose, and most preferably cross-linked acrylate copolymer.

The cellulosic component and absorbent fiber component optimally conjugated with the application of punching means to render thin and uniform non-woven fabric structure that has holding capacity or absorbency more than 1500%.

In an embodiment, the non-woven fabric structure has holding capacity or absorbency in the range of 1500% to 5500%, preferably 1500% to 5400%or 1500% to 5300% or 1500% to 5200% or 1500% to 5100%, or 1500% to 5000%, more preferably 1600% to 5500% or 1600% to 5400% or 1600% to 5300% or 1600% to 5200% or 1600% to 5100%, or 1600% to 5000%, even more preferably 1700% to 5500% or 1700% to 5400% or 1700% to 5300% or 1700% to 5200% or 1700% to 5100%, or 1700% to 5000%, even more preferably 1800% to 5500% or 1800% to 5400% or 1800% to 5300% or 1800% to 5200% or 1800% to 5100%, or 1800% to 5000%, even more preferably 1900% to 5500% or 1900% to 5400% or 1900% to 5300% or 1900% to 5200% or 1900% to 5100%, or 1900% to 5000%, and most preferably 1900% to 5000%,

The non-woven fabric structure has air permeability of more than 750 L/dm²/min at 200 Pa and bursting strength of more than 5.4kg/cm².

In an embodiment, the non-woven fabric structure has air permeability in the range of 750 to 1000 L/dm²/min at 200 Pa, preferably 760 to 990L/dm²/min at 200 Pa or 760 to 980 L/dm²/min at 200 Pa or 760 to 970 L/dm²/min at 200 Pa or 760 to 960 L/dm²/min at 200 Pa or 760 to 950 L/dm²/min at 200 Pa or 760 to 940 L/dm²/min at 200 Pa or 760 to 930 L/dm²/min at 200 Pa, more preferably 770 to 1000 L/dm²/min at 200 Pa or 770 to 990 L/dm²/min at 200 Pa or 770 to 980 L/dm²/min at 200 Pa or 770 to 970 L/dm²/min at 200 Pa or 770 to 960 L/dm²/min at 200 Pa or 770 to 950 L/dm²/min at 200 Pa or 770 to 940 L/dm²/min at 200 Pa or 770 to 930 L/dm²/min at 200 Pa, even more preferably 780 to 1000 L/dm²/min at 200 Pa or 780 to 990 L/dm²/min at 200 Pa or 780 to 980 L/dm²/min at 200 Pa or 780 to 970 L/dm²/min at 200 Pa or 780 to 960 L/dm²/min at 200 Pa or 780 to 950 L/dm²/min at 200 Pa or 780 to 940 L/dm²/min at 200 Pa or 780 to 930 L/dm²/min at 200 Pa, even more preferably 790 to 1000 L/dm²/min at 200 Pa or 790 to 990 L/dm²/min at 200 Pa or 790 to 980 L/dm²/min at 200 Pa or 790 to 970 L/dm²/min at 200 Pa or 790 to 960 L/dm²/min at 200 Pa or 790 to 950 L/dm²/min at 200 Pa or 790 to 940 L/dm²/min at 200 Pa or 790 to 930 L/dm²/min at 200 Pa, and most preferably 790 to 930 L/dm²/min at 200 Pa.

In an embodiment, the non-woven fabric structure has bursting strength in the range of 5.4 to 7.9kg/cm², more preferably 6.0 to 7.9 kg/cm².

The absorbent polymer source fiber component is present in the bi-component fabric in an amount greater than 1% by weight of total fabric core.

In an embodiment, the absorbent polymer source fiber component is present in the bi-component fabric in an amount in the range of 1% to 99% by weight of total fabric core, preferably in the range of 5% to 90% or 5% to 80% or 5% to 70% or 5% to 60% or 5% to 55% or 5% to 50% by weight of total fabric core, more preferably in the range of 6% to 90% or 6% to 80% or 6% to 70% or 6% to 60% or 6% to 55% or 6% to 50% by weight of total fabric core, even more preferably in the range of 7% to 90% or 7% to 80% or 7% to 70% or 7% to 60% or 7% to 55% or 7% to 50% by weight of total fabric core, even more preferably in the range of 8% to 90% or 8% to 80% or 8% to 70% or 8% to 60% or 8% to 55% or 8% to 50% by weight of total fabric core, even more preferably in the range of 9% to 90% or 9% to 80% or 9% to 70% or 9% to 60% or 9% to 55% or 9% to 50% by weight of total fabric core, even more preferably in the range of 10% to 90% or 10% to 80% or 10% to 70% or 10% to 60% or 10% to 55% or 10% to 50% by weight of total fabric core, and most preferably in the range of 10% to 50% or 10% to 40% by weight of total fabric core.

The ratio of the absorbent polymer source fiber to cellulosic fiber is in the range of 1:99 to 55:45.

In an embodiment, the ratio of the absorbent polymer source fiber to cellulosic fiber is in the range of 1:99, preferably in the range of 5:95 or 10:90 or 15:85, more preferably in the range of 20:80 or 25:75 or 30:70 or 35:65 or 40:60 or 45:55 or 50:50 or 55:45, and most preferably in the range of 40:60.

In another aspect, the presently claimed invention is directed to a method of manufacturing the non-woven fabric structure for liquid absorption. The steps of preparing the non-woven fabric structure comprising:
(a) opening bales of first fiber component cellulosic resource components and second fiber component i.e. absorbent polymer resource fiber component;
(b) mixing of the respective fiber components of step (a) in specific proportion to render bi-component at feed chute (1) apparatus;
(c) carding of bi-component fiber received in step (b) to render more fine-tuned fiber to grab and aligned bi-component fibers for performing combing, at cylinder (5) worker roller (6) assembly;
(d) cross-lapping of bi-component fiber received in step (c) to render partially oriented non-woven fabric structure by angularly disposed conveyers (10-11);
(e) drafting of bi-component fabric received in step (d) to render relatively thin and improved parallel alignment by differential roller assembly (6-7); and
(f) punching the bi-component fabric received in step (e) to render high entangled bi-component fabric by needle punching machine (12).

The steps for preparing non-woven fabric structure for liquid absorption further comprising:
(g) calendaring the bi-component fabric received from step (f) to render desired finishing in final product; and,
(h) inspecting the bi-component fabric received in step (f) to cross check quality control parameters.

In another aspect, the presently claimed invention is directed to an article comprising non-woven fabric structure as defined hereinabove.

The article can be baby pads, baby diapers, adult diapers, sanitary napkins, pet pads, puppy pads, and patient under pads.

In another aspect, the presently claimed invention is directed to use of the non-woven fabric structure as defined hereinabove for liquid absorption.

The present needle punched non-woven fabric structure is developed for the application as absorbent layer of hygiene products for example Sanitary napkin, Diapers, Puppy pads, Patient under pads and other relevant products for single or multiple use.

The properties of needle-punched fabrics depend on the length and characteristics of the fibers, the physical properties of the web, and the techniques used to produce the web. It is observed that needled fabrics lack any structural pattern because the needles punch and intermingle the fibers in such a random way that the fabric on the surface appears uniform.

The crux of the invention lies in providing novel and innovative needle punched nonwoven structure of GSM ranges from 50 to 1000 or more in combination of different fibers. The fiber could be natural fibers or from synthetic origin in appropriate ratio. Natural fiber could be viscose, cotton, kapok, nettle, wool, silk, ramie, hemp and synthetic fiber could be super absorbent polymer (SAP) fiber, regenerated cellulose fiber, polyester fiber, crosslinked acrylate copolymer, nylon fiber with circular or any other suitable cross-section. The Needle punched nonwoven structure (GSM 50 to 1000) made of SAP fiber and other cellulose fiber with different cross section, kapok, nettle for use of absorbent core.

The methodology and assembly of the present invention focuses on following parameter that lead to fulfil the objective of the present invention:
1. GSM range
2. Thickness range
3. Fiber blend ratio
4. Different types of fiber
5. Process and assembly parameter to optimized fabric structure

The essential steps that followed in the present invention is provided here that essentially clear the process parameter in the matter:-

### Opening> Mixing>Carding>Cross-lapping> Drafting>N eedle-punch> Additional Needle punch>Calendaring>Inspection

### 1. Opening:

"Opening" in textile processing typically refers to the initial stage in the manufacturing process where raw fibers, such as cotton, wool, or synthetic fibers, are loosened and separated. This process helps to prepare the fibers for subsequent processing steps such as carding, Cross-lapping, drafting. Opening can be achieved through various methods including mechanical methods such as or carding. The primary goal of opening is to create a uniform and homogeneous fiber mass that can be further processed efficiently.

### 2. Mixing:

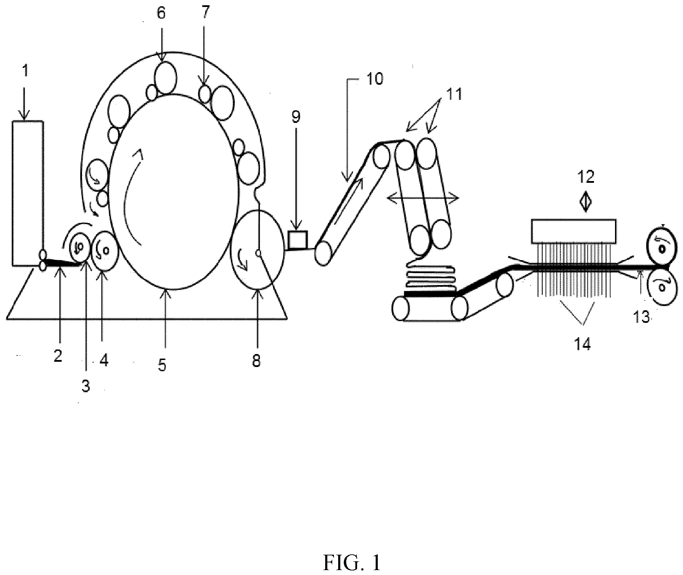

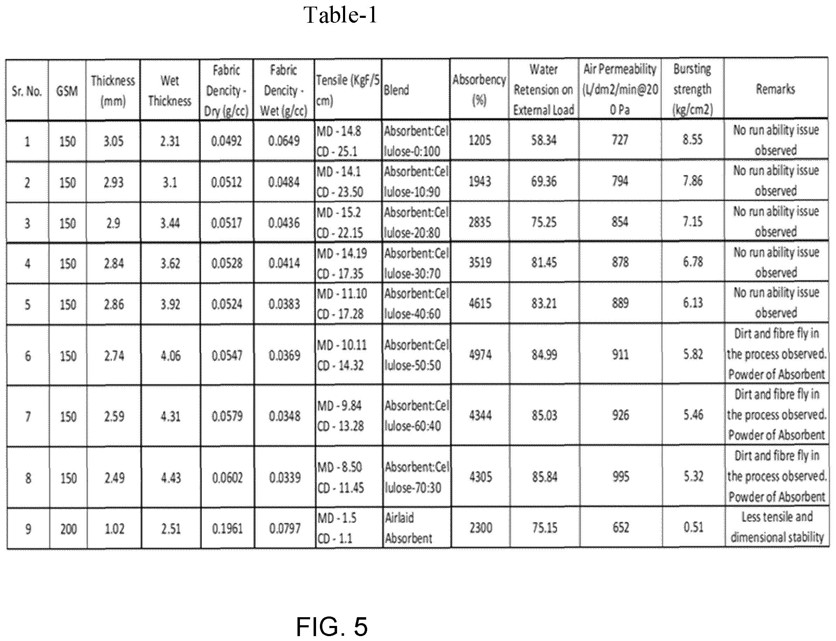

It relates to combining different types of fibers or materials to create blends with specific properties. The present step is done to achieve desired characteristics in the final textile product, such as strength, absorbency and softness. Mixing can involve blending natural fibers like cotton, wool, or silk with synthetic fibers such as absorption fibers or even combining different types of natural fibers i.e. regenerative cellulose fibers. Overall, mixing plays a crucial role in the present invention as the same allows user to create fabrics with a wide range of properties to meet various end-use requirements. Fig. 1 denotes feeding chute (1) where the specific proportion of bi-component fiber system (i.e. absorbent polymer resource and cellulosic) feed in order to mix the same in further proportion. The details of proportion given in tabular form (Table-1 shown in Fig. 5) of the present specification.

### 3. Carding:

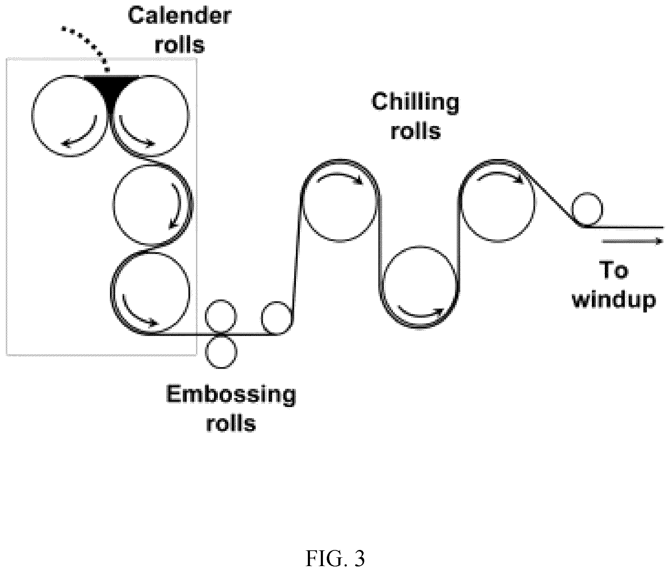

During carding, the fibers are passed through a series of rotating cylinders covered in fine wires or teeth, known as carding machines. These cylinders comb and separate the fibers, aligning them in the same direction. The representative figure depicted in Fig. 3 of present application. The carding plays vital role in combining initial fibers for absorbentfiber and regenerated cellulosic fibers. The Fig. 1 denotes the large cylinder at centre and closely conjugated worker roller and striper roller arrangement that essentially perform efficient carding to the bi-component system. A cylinder worker roller in the present invention refers to a rotating cylinder covered with fine teeth that is a key component of a carding machine, primarily responsible for grabbing and aligning bi-component fibers as they are fed through the machine, essentially performing the main carding action between the cylinder and the "worker roller" which actively pulls the fibers from the cylinder to further process them; it's a crucial part of preparing fibers for spinning into yarn. The cylinder acts as the main surface where fibers are initially deposited and then combed by the worker roller, which has a slightly different tooth arrangement to effectively grab and pull the fibers apart and align them. The said arrangement provides unique mixing and carding to the bi-component (absorbent and cellulosic fiber) system.

### 4. Cross lapping:

"Cross-lapping" is a technique used in textile manufacturing to create nonwoven fabrics. In this process, a web of fibers is formed by carding or other methods, and then it is passed through a series of conveyors that move in perpendicular or inclined directions. As the web moves across these conveyors, it is alternately laid down in one direction and then the other, creating a crisscross pattern. This results in a fabric with improved strength, uniformity, and dimensional stability. Cross-lapping is used in the production of nonwoven fabrics for applications such as filtration, insulation, and geotextiles. Fig. 1 also denotes cross lapping wherein inclined conveyer and pendulum conveyer provides cross lapping performed to bi-component fiber to further process.

### 5. Drafting:

The present step is crucial process in the present invention, particularly in spinning. It involves the attenuation and elongation of the fiber strand to achieve the desired yarn thickness and consistency.

During drafting, the bi-component fibers are pulled and elongated to reduce their thickness and improve their parallel alignment. This process is typically achieved using drafting rollers or pairs of rollers, where the front roller rotates faster than the back roller, creating tension and elongating the fibers. As the fibers pass through the drafting zone, they are attenuated and drawn out to the desired level of thinness.

### 6. Needle punch

The present steps are a mechanical process used in the production of nonwoven fabrics. It involves punching barbed needles repeatedly through a web of fibers to entangle and interlock them, creating a cohesive fabric structure.

During needle punching, the web of fibers is placed on a bed or conveyor, and a needle-punching machine with rows of barbed needles is passed over the web. As the needles penetrate the fibers, they pull and intertwine them, effectively bonding the fibers together. The depth and frequency of needle penetration can be adjusted to control the fabric's density, thickness, and strength.

The present process steps has optimized to provide thin, uniform and robust intermediate product by providing specific needle specification that readily punch the two different composition which were difficult to intermingle the fibers in such a random way that the fabric on the surface appears uniform.

### 7. Additional Needle Punching:

In order to align with objects of the present invention additional needle punching machine is provided within the assembly line of the present machine assembly. The present invention provides crux by combining advanced engineering with versatility and efficiency, Quadra Needle punch technology is ultimate for producers want to have high-quality, cost-effective, and sustainable nonwoven solutions:
- Capable of handling a wide range of fibers, including synthetic, natural, and recycled materials
- Delivers superior blend and surface uniformity, enhancing the appearance and functionality of the final product
- Features a quad configuration (four needle boards) that ensures even and consistent fiber entanglement
- Improves fabric strength, durability, and uniformity across the entire width
- Enables precise control over parameters like density, thickness, and surface finish
- Additional nonwoven layers of different types can be joined.

The present invention discloses the Quadra Needle punch technology in most preferred embodiment, it can be made with any needle punch manufacturing machine including, but not limited to, Single and Double board needle looms, Tandem needle looms, Velour, Structuring, and Patterning Needle Looms, available in the market provided uniform blend mixing and fabric quality. The said assembly is provided in Fig. 3 of present application. The said assembly provides effective and rapidly controlled penetration from top and bottom of the bi-component web.

### 8. Calendaring

The process of calendaring followed by needle punching operations.

A calendaring process is a finishing operation used in present invention, to impart specific properties to materials by passing them through a series of heated rollers under significant pressure. Here's how the calendaring process typically works:

### Step 1. Material Preparation

The material to be calendared, such as fabric, is prepared and fed into the calendaring machine. Before entering the calendaring section, the material may undergo pre-treatment processes such as drying, softening, or impregnation to prepare it for calendaring.

### Step 2. Roller Arrangement

The calendaring machine consists of a series of cylindrical rollers, typically made of steel or other hard materials, arranged vertically or horizontally. The number of rollers can vary depending on the desired effect and the material being processed.

### Step 3. Passing through Calendaring Rolls

The material is then passed through a series of closely spaced, hard-surfaced rollers or cylinders, known as calendar rolls. These rolls can be heated or cooled, depending on the material and the desired outcome. Furthermore, as the material passes through the nips (the points where the rolls are in contact with each other), it is subjected to high pressure. This pressure compresses the material, reducing its thickness and increasing its density.

### Step 4. Controlling the Rolls

The speed, temperature, and gap between the rolls can be adjusted to control the degree of calendering. This allows for precise control over the final properties of the material, such as smoothness, gloss, and thickness. The heat generated by the friction between the rolls and the material, along with the heat from the rolls themselves if they are heated, helps to soften the material, making it more pliable and easier to compress.

### 9. Inspection

The said step refers to the process of examining textile products at various stages of production to ensure they meet specified quality standards. This can include visual inspection, physical testing, and measurement of critical parameters.

During inspection, trained personnel or automated systems evaluate the textile products for defects such as irregularities in color, pattern, texture, or stitching. They also check for flaws like holes, tears, stains, or unevenness in the final product. In addition to visual inspection, various tests may be conducted to assess properties such as tensile strength, abrasion resistance, shrinkage, color fastness, and dimensional stability.

Inspection is crucial for maintaining product quality and consistency, as well as for identifying and addressing any issues or defects early in the production process. It helps to ensure that the final textile products meet customer requirements and regulatory standards.

The present process utilizes optimized blend of Absorbent fiberin combination with regenerated cellulose fiber of various cross-section. In preferred embodiment of the present invention, the output of the process is the Multilayer combination of the various fabric component prepared individually.

In another embodiment the present invention provides output as thin single layer composition prepared by needle punching two different composition i.e. Absorbent fiber and regenerative cellulose fiber.

It is submitted that the present invention provides easy adaptability to versatile solution of preparing different types of fabric for different end user application. In the practical approach the present invention supports variability in the ratio of 1:99 for absorbent fiber to regenerative cellulose fibers. Further in specific embodiment, the different GSM product in the range of 50 to 1000 or more.

The methodology and assembly of the present needle punched structure provides air permeability, bursting strength, and tensile strength in machine and cross machine direction that is more than sufficient to increase the speed of final product conversion at the same time the water holding capacity is similar or exceeding the required for such application.

In order to provide significance for particular % of fabric composition for Absorbent fiber and regenerative fibers that serves the object of liquid absorption in the range of 0:100 and vice-versa the experiment carried out.

In order to have optimised ratio absorbency testing are done with Deionized water.

Fig. 5 shows Table-1 showing experimental data for testing data collected for various ratio of absorption fiber and cellulosic fiber composition.

In order to achieve the object of the present invention trials were conducted by varying the mixing percentage of absorbent fibers from 0% to 70%, with the remaining component being cellulosic fibers. It is evident from the trial that there is significant increase in the absorbency of the material as the proportion of absorbent fibers increased, particularly up to 50%. This improvement is attributed to the absorbent fibers' capacity to retain liquids. However, when the absorbent fiber content exceeded 50%, the rate of absorbency increment achieves plateau. This plateau effect is due to gel blocking, where the swollen fibers trap liquid in localized areas, hindering further liquid distribution and absorption across the material. These observations suggest that at 50% proportion of absorbent fibers represent an optimal balance for maximizing absorbency without encountering the limitations caused by gel blocking.

Fiber dirt and fly generation became noticeable above 40% absorbent fiber content. At 50% and above, the generation of dirt and fly reached unacceptable levels, posing challenges for material handling and processing. This suggests that while the absorbency benefits peak around 50% absorbent fiber content, the associated operational drawbacks must be carefully considered to determine the most feasible composition for practical applications.

The absorbency of regular needle punch fabrics composed of natural, regenerated, and synthetic fibers typically ranges between 500% and 1500%. This range is achieved by maintaining consistent parameters such as GSM (grams per square meter), thickness, and specific density. Among these factors, following factors are critical to determine absorbency performance:
- Thickness
- Specific density

Thicker needle punch fabrics with lower specific density have a tendency to exhibit higher absorbency capacity. This is because increased thickness provides more space within the fabric structure for liquid retention, while a lower specific density enhances the porosity, allowing the fabric to absorb and hold more liquid effectively.

While increasing the thickness and reducing the specific density of needle punch fabrics enhance absorbency, these changes have a trade-off in terms of strength and dimensional stability. Thicker fabrics with lower specific density are inherently less compact, leading to reduced fiber entanglement and weaker mechanical bonding within the structure. This compromises the fabric's tensile strength and ability to maintain its dimensions under stress.

The balance of thickness, specific density, fabric durability and stability is critical for optimizing the performance of needle punch fabrics in applications where both absorbency and structural integrity are essential.

In needle punch fabrics with higher thickness and lower dimensional stability, the reduction in thickness upon water absorption is significantly higher compared to fabrics with a more compact structure. This phenomenon occurs because the loosely packed fibers in less stable fabrics allow for greater compression and deformation when exposed to liquid. The collapse of the fabric's structure under the weight of absorbed water reduces its ability to retain liquid, effectively limiting its maximum absorbency.

As a result, despite the potential for high porosity and absorbency in such fabrics, their absorbency capacity is restricted to a maximum of approximately 1500% of their weight. This limitation highlights the importance of optimizing the balance between fabric thickness, specific density, and structural stability to ensure both high absorbency and consistent performance in practical applications.

These findings highlight the importance of optimizing fabric design parameters to achieve the desired balance of absorbency and structural integrity.

Absorbent fibers possess exceptional water-holding capabilities, absorbing up to 95 times their weight under a load of 0.30 PSI. This unique property significantly enhances the absorbency of needle punch fabrics, surpassing the limitations of conventional natural, regenerated, and synthetic fibers.

When incorporated into the fabric structure, these fibers not only increase the overall liquid retention capacity but also enable the fabric to maintain its absorbency performance under pressure. This is particularly advantageous in applications where the fabric is subjected to compressive forces, as the absorbent fibers mitigate the reduction in absorbency typically caused by structural collapse.

It is observed that by integrating absorbent fibers into needle punch fabrics, the present invention achieves object pertaining to advanced material performance, including superior liquid retention and functional adaptability, making them suitable for high-demand applications in hygiene, medical, and industrial sectors.

In one of the preferred embodiments for commercial production, 40% content of absorbent fiber can be selected.

### Process parameter consideration:

Fiber dirt and fly generation became noticeable above 40% absorbent fiber content. At 50% and above, the generation of dirt and fly reached unacceptable levels, posing challenges for material handling and processing.

When the absorbent fiber content exceeded 50%, the rate of absorbency increase leveled off. This plateau effect is due to gel blocking, where the swollen fibers trap liquid in localized areas, hindering further liquid distribution and absorption across the material. These findings suggest that a 50% proportion of absorbent fibers represents an optimal balance for maximizing absorbency without encountering the limitations caused by gel blocking.

Looking the above issues, 40% content of absorbent fiber can be considered for commercial production.

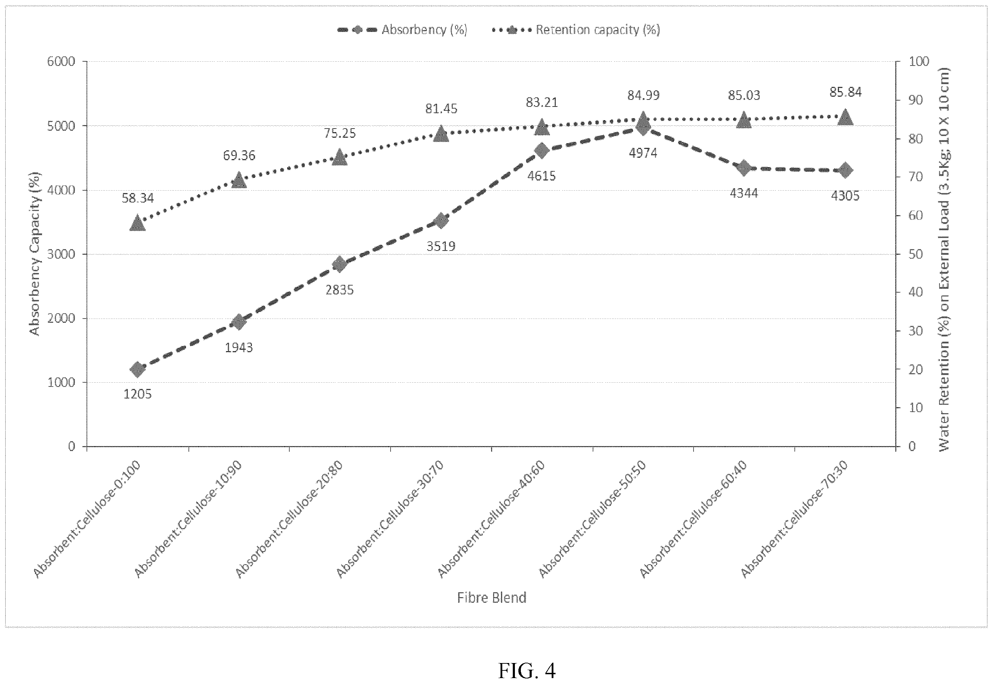

Fig. 4 depicts the crucial aspect of data for selecting optimized fiber ratio is given as graph as Composition blend Vs. Absorbency in %. The said graph justify the selection of ratio for absorption fiber and cellulosic fibers.

The present invention provides following advantages:
1. Single layer needle punched non-woven structured fabric with improved mechanical properties
2. High liquid holding capacity
3. Production friendly - High conversion speed of final product conversion
4. Improved production performance during final product formation
5. Higher Strength in both machine and cross machine direction

A detailed description of one or more embodiments of the invention is provided herein along with accompanying figures that illustrate the principles of the invention. The invention is described in connection with such embodiments, but the invention is not limited to any embodiment. The scope of the invention is limited only by the claims and the invention encompasses numerous alternatives, modifications and equivalents. Numerous specific details are set forth in the description herein in order to provide a thorough understanding of the invention. These details are provided for the purpose of example and the invention may be practiced according to the claims without some or all of these specific details. For the purpose of clarity, technical material that is known in the technical fields related to the invention has not been described in detail so that the invention is not unnecessarily obscured.

For the sake of clarity, processes and methods herein may have been illustrated with a specific flow, but it should be understood that other sequences may be possible and that some may be performed in parallel, without departing from the spirit of the invention. Additionally, steps may be subdivided or combined. It will be understood by one of ordinary skill that an embodiment can contain an alternate order of the steps, or an alternate configuration or arrangement of elements, adapted to a particular application disclosed herein. All such variations and modifications are intended to fall within the scope of this disclosure. The depiction and description of steps in any particular order or elements in any particular arrangement is not intended to exclude embodiments having the steps in a different order or elements in a different arrangement, unless required by a particular application, explicitly stated, otherwise clear from the context, or unless the order or arrangement would render an embodiment inoperative. Although the present invention has been described above in terms of specific embodiments, it is anticipated that alterations and modifications to this invention will no doubt become apparent to those skilled in the art and may be practiced within the scope and equivalents of any appended claims.

Benefits, features, and advantages of the present invention, in addition to the structure and arrangement of various embodiments of the present invention, are described in detail herein, with reference to the accompanying drawings. Note that the embodiments of the invention disclosed herein are illustrative and explanatory of the invention, and do not limit the invention to those specific embodiments disclosed. Those with ordinary skill in the relevant art(s) will recognize additional embodiments of the invention beyond those disclosed herein, in view of what is commonly known in the art(s) and the teaching herein.

While multiple embodiments are disclosed, still other embodiments of the present invention will become apparent to those skilled in the art from this detailed description. The invention is capable of myriad modifications in various obvious aspects, all without departing from the scope of the present invention. Accordingly, the drawings and descriptions are to be regarded as illustrative in nature and not restrictive.

As used herein, the singular form "a", "an" and "the" include plural references unless the context clearly dictates otherwise. For example, the term "a compound" or "at least one compound" may include a plurality of compounds, including mixtures thereof.

While, the invention has been described with respect to the given embodiment, it will be appreciated that many variations, modifications and other applications of the invention may be made. However, it is to be expressly understood that such modifications and adaptations are within the scope of the present invention, as set forth in the following claims.

### Reference:

- Feed Chute (1)
- Feed Material (2)
- Feed Roller (3)
- Licker in (4)
- Cylinder (5)
- Worker roller (6)
- Stripper roller (7)
- Doffer (8)
- Web Take-off (9)
- Inclined Conveyer (10)
- Pendulum Conveyer (11)
- Reciprocating Needle board (12)
- Fabric (13)
- Needle (14)

Some embodiments of the invention can be described using the following clauses:
1. A non-woven fabric structure for liquid absorption comprising,
   at least one fabric core structure containing at least two different fiber components,
      (i) first fiber component as cellulosic resource to increase comfort feel during the application; and,
      (ii) second fiber component as absorbent polymer resource fiber component to increase water absorption during the application,
   wherein,
   said cellulosic fiber component and absorbent polymer resource fiber component combined together such that a large portion of fabric core have uniformly distributed conjugated bi-component,
   wherein,
   the absorbent fiber preferably may have particulate polymer material discontinuously distributed on total fabric core, and;
   the first fiber component and second fiber component are combined together such that the each component entangled with another component in uniform way to render fabric core.
2. The non-woven fabric structure for liquid absorption as described in clause 1, wherein the cellulosic fiber is selected from a group consisting of viscose, cotton, linen, hemp, jute, kapok, ramie, sisal, bamboo, coconut, and mixtures of two or more thereof.
3. The non-woven fabric structure for liquid absorption as described in clause 1, wherein the absorbent polymer fiber component is selected from a group consisting of polyacrylamide copolymer, ethylene maleic anhydride copolymer, cross-linked carboxy methylcellulose, polyvinyl alcohol copolymers, cross-linked acrylate copolymer, cross-linked polyethylene oxide, starch grafted copolymer of polyacrylonitrile, and mixtures of two or more thereof.
4. The non-woven fabric structure for liquid absorption as described in clause 1, wherein the cellulosic component and absorbent fiber component optimally conjugated with the application of punching means to render thin and uniform non-woven fabric structure that has holding capacity or absorbency more than 1500%.
5. The non-woven fabric structure for liquid absorption as described in clause 1, wherein the non-woven fabric structure has air permeability of more than 750 L/dm²/min at 200 Pa and bursting strength of more than 5.4kg/cm².
6. The non-woven fabric structure for liquid absorption as described in any of clauses 1 to 5, wherein the absorbent polymer source fiber component is present in the bi-component fabric in an amount greater than 1% by weight of total fabric core.
7. The non-woven fabric structure for liquid absorption as described in any of clauses 1 to 6, wherein the ratio of the absorbent polymer source fiber to cellulosic fiber is in the range of from 1:99 to 55:45.
8. A method of manufacturing the non-woven fabric structure for liquid absorption as described in any of clauses 1 to 7, wherein the steps of preparing the non-woven fabric structure comprising:
   (a) opening bales of first fiber component cellulosic resource components and second fiber component i.e. absorbent polymer resource fiber component;
   (b) mixing of the respective fiber components of step (a) in specific proportion to render bi-component at feed chute (1) apparatus;
   (c) carding of bi-component fiber received in step (b) to render more fine-tuned fiber to grab and aligned bi-component fibers for performing combing, at cylinder (5) worker roller (6) assembly;
   (d) cross-lapping of bi-component fiber received in step (c) to render partially oriented non-woven fabric structure by angularly disposed conveyers (10-11);
   (e) drafting of bi-component fabric received in step (d) to render relatively thin and improved parallel alignment by differential roller assembly (6-7); and
   (f) punching the bi-component fabric received in step (e) to render high entangled bi-component fabric by needle punching machine (12).
9. The method of manufacturing the non-woven fabric structure for liquid absorption as described in clause 8, wherein the steps for preparing non-woven fabric optionally comprising:
   (g) calendaring the bi-component fabric received from step (f) to render desired finishing in final product; and,
   (h) inspecting the bi-component fabric received in step (f) to cross check quality control parameters.
10. An article comprising non-woven fabric structure as described in any of clauses 1 to 7.
11. The article as described in any of clause 10, wherein the article can be baby pads, baby diapers, adult diapers, sanitary napkins, pet pads, puppy pads, and patient under pads.

## Claims

1. A non-woven fabric structure for liquid absorption comprising,
at least one fabric core structure containing at least two different fiber components,
(i) a first fiber component as a cellulosic resource fiber component to increase comfort feel during the application; and
(ii) a second fiber component as an absorbent polymer resource fiber component to increase water absorption during the application,
wherein:
the cellulosic resource fiber component and the absorbent polymer resource fiber component are combined together such that a large portion of a fabric core has a uniformly distributed conjugated bi-component,
the absorbent polymer resource fiber optionally has particulate polymer material discontinuously distributed on total of the fabric core, and
the first fiber component and the second fiber component are combined together such that each component is entangled with another component in a uniform way to render the fabric core.

2. The non-woven fabric structure for liquid absorption as claimed in claim 1, wherein the cellulosic resource fiber component is selected from a group consisting of viscose, cotton, linen, hemp, jute, kapok, ramie, sisal, bamboo, coconut, and mixtures of two or more thereof.

3. The non-woven fabric structure for liquid absorption as claimed in claim 1 or 2, wherein the absorbent polymer fiber component is selected from a group consisting of polyacrylamide copolymer, ethylene maleic anhydride copolymer, cross-linked carboxy methylcellulose, polyvinyl alcohol copolymers, cross-linked acrylate copolymer, cross-linked polyethylene oxide, starch grafted copolymer of polyacrylonitrile, and mixtures of two or more thereof.

4. The non-woven fabric structure for liquid absorption as claimed in any of claims 1 to 3, wherein the cellulosic resource fiber component and the absorbent polymer fiber component are optimally conjugated with an application of punching means to render thin and uniform non-woven fabric structure that has holding capacity or absorbency more than 1500%.

5. The non-woven fabric structure for liquid absorption as claimed in any of claims 1 to 4, wherein the non-woven fabric structure has an air permeability of more than 750 L/dm²/min at 200 Pa and a bursting strength of more than 5.4kg/cm².

6. The non-woven fabric structure for liquid absorption as claimed in any of claims 1 to 5, wherein the absorbent polymer source fiber component is present in the bi-component fabric in an amount greater than 1% by weight of total fabric core.

7. The non-woven fabric structure for liquid absorption as claimed in any of claims 1 to 6, wherein the ratio of the absorbent polymer source fiber to cellulosic fiber is in the range of from 1:99 to 55:45.

8. A method of manufacturing the non-woven fabric structure for liquid absorption as claimed in any of claims 1 to 7, wherein the steps of preparing the non-woven fabric structure comprise:
(a) opening bales of the first fiber component being the cellulosic resource fiber component and the second fiber component being the absorbent polymer resource fiber component;
(b) mixing the respective fiber components of step (a) in specific proportion to render a bi-component fiber at feed chute (1) apparatus;
(c) carding the bi-component fiber received in step (b) to render a more fine-tuned fiber to grab and align the bi-component fibers for performing combing, at a cylinder (5) worker roller (6) assembly;
(d) cross-lapping the bi-component fiber received in step (c) to render a partially oriented non-woven fabric by angularly disposed conveyers (10-11);
(e) drafting the bi-component fiber received in step (d) to render relatively thin and improved parallel alignment by differential roller assembly (6-7); and
(f) punching the bi-component fiber received in step (e) to render a high entangled bi-component fabric by needle punching machine (12).

9. The method of manufacturing the non-woven fabric structure for liquid absorption as claimed in claim 8, wherein the steps for preparing non-woven fabric structure comprise:
(g) calendaring the bi-component fabric received from step (f) to render desired finishing in final product; and,
(h) inspecting the bi-component fabric received in step (f) to cross check quality control parameters.

10. An article comprising the non-woven fabric structure as claimed in any of claims 1 to 7.

11. The article as claimed in claim 10, wherein the article is baby pads, baby diapers, adult diapers, sanitary napkins, pet pads, puppy pads, or patient under pads.
